(19) Europäisches Patentamt
European Patent Office
Office européen des brevets

(11) Numéro de publication: **0 485 299 B1**

(12) # FASCICULE DE BREVET EUROPEEN

(45) Date de publication de fascicule du brevet: **14.09.94**

(51) Int. Cl.5: **A61K 7/48**, A61K 7/00, A61K 7/06, A61K 7/02

(21) Numéro de dépôt: **91403016.8**

(22) Date de dépôt: **08.11.91**

(54) **Composition cosmétique anhydre sous forme de mousse aérosol.**

(30) Priorité: **09.11.90 FR 9013948**

(43) Date de publication de la demande:
**13.05.92 Bulletin 92/20**

(45) Mention de la délivrance du brevet:
**14.09.94 Bulletin 94/37**

(84) Etats contractants désignés:
**AT BE CH DE ES FR GB IT LI NL**

(56) Documents cités:
**FR-A- 2 157 784**
**FR-A- 2 192 795**
**FR-A- 2 647 445**
**US-A- 3 419 658**

(73) Titulaire: **L'OREAL**
**14, Rue Royale**
**F-75008 Paris (FR)**

(72) Inventeur: **Louvet, Nathalie**
**13, rue du Guesclin**
**F-94240 L'Hay les Roses (FR)**
Inventeur: **Lukassen, Liliane**
**Résidence de l'Hermitage,**
**24, Allée d'Alsace**
**F-94550 Chevilly Larue (FR)**
Inventeur: **Yquel, Jean-Pierre**
**134, rue d'Estienne d'Orves**
**F-92700 Colombes (FR)**

(74) Mandataire: **Stalla-Bourdillon, Bernard**
**CABINET NONY & CIE**
**29, rue Cambacérès**
**F-75008 Paris (FR)**

EP 0 485 299 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet une composition cosmétique anhydre sous forme aérosol pour la formation d'une mousse, celle-ci étant tout particulièrement destinée au démaquillage du visage et des yeux, aux soins de la peau en particulier des peaux sèches ou au traitement des cheveux.

Le principal problème que l'on rencontre dans la préparation de compositions aérosols pour la formation d'une mousse réside essentiellement dans le fait que la mousse une fois formée doit présenter une bonne stabilité pendant un certain laps de temps.

Par ailleurs, il convient que la mousse soit suffisamment ferme et onctueuse à l'application.

L'état de la technique est essentiellement constitué par le brevet français n° 2.157.784 qui décrit une composition anhydre aérosol contenant un agent moussant, un liquide organique moussant, une résine de silicone et un propulseur. Le but recherché dans ce brevet est d'obtenir une mousse stable mais qui se "brise facilement" et l'addition de silicones permet de l'atteindre.

La présente invention permet de réaliser des compositions anhydres sous forme aérosol pour la formation d'une mousse dont la qualité de la mousse présente à la fois de bonnes caractéristiques de stabilité, de fermeté et d'onctuosité.

Après diverses études on a en effet constaté de façon inattendue et surprenante qu'une certaine classe de composés dérivés des acides carboalkyloxy amino-11 undécanoïques ou de leurs esters, constituait d'excellents agents moussants pour des compositions sous forme aérosol. Grâce à l'emploi de ces composés, il est ainsi possible d'obtenir des mousses présentant une excellente stabilité dans le temps ainsi que par ailleurs de très bonnes propriétés cosmétiques notamment d'onctuosité.

Du fait de cette stabilité améliorée, les produits sont d'une application facile et d'une consistance agréable à l'utilisation.

La présente invention a donc pour objet à titre de produit industriel nouveau une composition cosmétique anhydre sous forme aérosol pour la formation d'une mousse constituée d'un propulseur et d'une phase grasse contenant en association au moins une huile cosmétique ou un mélange d'une huile et d'un corps gras et au moins un agent moussant, ledit agent moussant correspondant à la formule générale suivante :

$$R_1-O-\underset{\underset{O}{\|}}{C}-NH-(CH_2)_{10}-\underset{\underset{O}{\|}}{C}-OR \qquad (I)$$

dans laquelle
R représente un atome d'hydrogène ou un radical alkyle ayant de 14 à 20 atomes de carbone, et
$R_1$ représente un radical alkyle ayant de 8 à 18 atomes de carbone.

Selon une forme de réalisation particulière le radical R représente de préférence un radical alkyle ayant de 16 à 18 atomes de carbone et le radical $R_1$ un radical alkyle ayant de 10 à 16 atomes de carbone.

Parmi les radicaux alkyles ayant de 14 à 20 atomes de carbone on peut notamment citer les suivants : tétradécyle, hexadécyle et octadécyle, le radical hexadécyle étant particulièrement préféré.

Parmi les radicaux alkyles ayant de 8 à 18 atomes de carbone on peut citer les suivants : octyle, décyle, dodécyle, tétradécyle, hexadécyle, hexyl-2-décyle et isostéaryle, les radicaux décyle et hexadécyle étant particulièrement préférés.

Parmi les agents moussants de formule générale (I) ci-dessus on peut notamment citer les suivants :
l'acide N-carbohexadécyloxy amino-11 undécanoïque,
l'acide N-carbodécyloxy amino-11 undécanoïque,
le N-carbohexadécyloxy amino-11 undécanoate d'hexadécyle,
le N-carbohexadécyloxy amino-11 undécanoate d'octadécyle, et
le N-carbodécyloxy amino-11 undécanoate d'hexadécyle.

Les acides N-carboalkyloxy amino-11 undécanoïques sont pour la plupart connus par contre leurs esters sont nouveaux.

Diverses méthodes peuvent être employées pour leur obtention ; l'une consiste à faire réagir un alcool gras ($R_1OH$) sur un isocyanate de formule :

$$O = C = N - (CH_2)_{10} - \overset{\overset{\displaystyle O}{\|}}{C} - OR \qquad (II)$$

R et $R_1$ ayant les mêmes significations que celles données ci-dessus pour la formule (I).

Comme autre méthode on peut utiliser celle consistant à faire réagir un chloroformiate de formule $R_1OCOCl$ ou un azolide de formule :

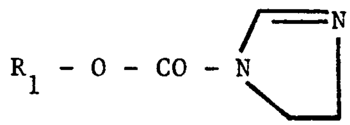

sur une amine de formule :

$$H_2N - (CH_2)_{10} - \overset{\overset{\displaystyle O}{\|}}{C} - OR \qquad (III)$$

R et $R_1$ ayant la même signification que celle donnée ci-dessus.

Ces deux méthodes qui ont pour objet la formation de la fonction carbamate, peuvent être mises en oeuvre selon les méthodes conventionnelles telles que décrites dans la littérature et en particulier dans le document "Advanced Organic Chemistry" Third Edition, Ed. Jerry, March 1985.

Les réactions sont généralement conduites en milieu solvant organique et/ou aqueux en présence d'une base de préférence la soude, la potasse ou la triéthylamine.

Ces méthodes peuvent être également mises en oeuvre à partir des acides des composés (II) et (III) (R = H), auquel cas on accède aux esters selon les méthodes classiques en particulier par estérification en présence de l'alcool choisi, en excès ou non, et d'un catalyseur acide tel que l'acide sulfurique ou l'acide para-toluène sulfonique éventuellement dans un solvant organique de préférence un solvant aromatique tel que le toluène.

A partir des sels de ces acides, il est également possible d'accéder aux esters selon les méthodes classiques en particulier par substitution des halogénures ou des sulfonates d'alcoyle, en milieu solvant organique ou par transfert de phase.

On peut également obtenir les esters par transestérification à partir des esters méthyliques et éthyliques correspondants et l'alcool souhaité.

Comme mentionné ci-dessus, les compositions cosmétiques anhydres selon l'invention contiennent au moins une huile ou un mélange d'au moins une huile et d'un corps gras.

Parmi les huiles susceptibles d'être utilisées dans les compositions selon l'invention, celles ci sont d'origine végétale, animale, minérale ou synthétique, et on peut en particulier citer :

- les huiles minérales telles que l'huile de paraffine, l'huile de vaseline et les huiles minérales ayant un point d'ébullition compris entre 310 et 410 °C,
- les huiles d'origine animale telles que le perhydrosqualène, l'huile de porc, l'huile de cheval, l'huile de tortue,
- les huiles végétales telles que l'huile d'amande douce, l'huile de calophyllum, l'huile de palme, l'huile d'avocat, l'huile de jojoba, l'huile de sésame, l'huile de tournesol, l'huile de karité, l'huile de carthame, l'huile de coprah, l'huile d'olive, l'huile de ricin, les huiles de germes de céréales telles que l'huile de germes de blé,
- les huiles de silicone telles que le diméthylpolysiloxane,
- les esters de synthèse tels que l'huile de Purcelin, le myristate de butyle, le myristate d'isopropyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate de décyle, le laurate d'hexyle, le di-caprylate de propylèneglycol, l'adipate de di-isopropyle,
- les alcools organiques tels que l'alcool oléïque, l'alcool linoléique, l'alcool linolénique, l'alcool isostéarylique, l'octyl-2-dodécanol, l'alcool isocétylique,
- les esters dérivés d'acide lanolique tels que le lanolate d'isopropyle, le lanolate d'isocétyle.

- On peut également citer : les acétyl-glycérides, les octanoates et décanoates d'alcools et de polyalcools tels que ceux de glycol et de glycérol, les ricinoléates d'alcools et de polyalcools tels que celui de cétyle.

Parmi les corps gras qui peuvent être utilisés en mélange avec au moins une huile on peut notamment mentionner :

- les cires minérales telles que les cires microcristallines, la paraffine, le petrolatum, la vaseline,
- les cires fossiles telles que l'ozokérite, la cire de montan,
- les cires d'origine animale telles la cire d'abeille, le spermacéti, la cire de lanoline, les dérivés issus de la lanoline tels que les alcools de lanoline, la lanoline hydrogénée, la lanoline hydroxylée, la lanoline acétylée, les acides gras de la lanoline, l'alcool de lanoline acétylée,
- les cires d'origine végétale telles que la cire de candelilla, la cire de carnauba, la cire du japon, le beurre de cacao,
- les huiles hydrogénées concrètes à 25°C telles que l'huile de ricin hydrogénée, l'huile de palme hydrogénée, le suif hydrogéné, l'huile de coco hydrogénée,
- les cires synthétiques telles que les cires de polyéthylène, les cires de polyéthylène copolymérisées,
- les esters gras concrets à 25°C tels que le mono-myristate de propylèneglycol, le myristate de myristyle,
- les cires de silicone telles que le poly(diméthylsiloxy) stéaroxysiloxane,
- parmi les cires on peut également citer : l'alcool cétylique, l'alcool stéarylique, les mono, di et triglycérides concrets à 25°C, le monoéthanolamide stéarique, la colophane et ses dérivés tels que les amiétates de glycol et de glycérol, les sucroglycérides et les oléates, myristates, lanolates, stéarates et dihydroxystéarates de calcium, magnésium, zinc et aluminium.

Selon l'invention la composition cosmétique anhydre contient de 20 à 99,95 % en poids et de préférence de 25 à 85 % en poids d'au moins une huile ou d'un mélange d'une huile et d'un corps gras, par rapport au poids total de la phase grasse.

La concentration en agent moussant de formule (I) est généralement comprise entre 0,05 et 20 % en poids et de préférence entre 0,2 et 5 % en poids par rapport au poids de la phase grasse.

Si l'on souhaite que la mousse après application puisse être éliminée par simple rinçage à l'eau, la composition devra alors de façon préférée contenir un ou plusieurs agent(s) tensioactif (s) oléosoluble(s) à une concentration de préférence comprise entre 5 et 60 % en poids par rapport au poids de la phase grasse.

De préférence l'agent tensioactif est du type nonionique et parmi ceux-ci, on peut citer les esters de polyols et de sucres, les produits de condensation de l'oxyde d'éthylène sur des acides gras, sur des alcools gras, sur des alcoylphénols à longue chaîne, sur des amides à longue chaîne, sur des esters de sorbitan, ou des alcools gras polyglycérolés ou encore des lécithines.

Parmi ces agents tensioactifs ceux particulièrement préférés sont : l'oléate de sorbitan, le trioléate de sorbitan, le tétraoléate de sorbitan, l'oléate de sorbitan oxyéthyléné à l'aide de 40 moles d'oxyde d'éthylène, le trioléate de sorbitan oxyéthyléné à l'aide de 20 moles d'oxyde d'éthylène, la lécithine de soja.

La phase grasse peut également contenir divers ingrédients cosmétiques oléosolubles choisis parmi les vitamines, les extraits végétaux ou animaux, les agents conservateurs, les parfums, etc...

Cette phase grasse est pressurisée à l'aide d'un propulseur représentant de 1 à 20 % en poids par rapport au poids total de la composition aérosol et de préférence de 3 à 15 %.

Parmi les propulseurs utilisables dans les compositions aérosols selon l'invention, on peut notamment mentionner le dioxyde de carbone, le protoxyde d'azote, l'air comprimé, l'azote, les hydrocarbures aliphatiques liquéfiables tels que le propane, les butanes dont l'isobutane, le pentane, l'isopentane, le néopentane et leurs mélanges. On peut également employer des hydrocarbures halogénés tels que le 1,1-difluoroéthané, le dichlorotétrafluoroéthane, le dichlorodifluorométhane, le monochlorodifluoroéthane et le monochlorodifluorométhane ainsi que leurs mélanges et en particulier le mélange de dichlorotétrafluoroéthane-dichlorodifluorométhane (40/60) ainsi que le mélange de monochlorodifluoroéthane et de monochlorodifluorométhane (60/40).

La proportion de propulseur utilisée n'est pas critique mais elle détermine la densité de la mousse produite. Plus la proportion de propulseur est élevée, plus la densité de la mousse est faible. Généralement, les densités de mousse sont situées dans le domaine d'environ 0,02 à environ 0,20 g/cm$^3$ et de préférence d'environ 0,05 à environ 0,15 g/cm$^3$.

ETUDES COMPARATIVES

Afin de mettre en évidence le choix particulier des composés de formule générale (I) comme agents moussants des compositions selon l'invention, on a comparé les composés A, B et C correspondant à la formule générale (I) aux composés de référence C à G ci-dessous, en ce qui concerne d'une part la masse volumique de la mousse après expansion à l'air et d'autre part la stabilité de la mousse dans le temps.

Composé A : $C_{16}H_{33}-O-\underset{\underset{O}{\|}}{C}-NH-(CH_2)_{10}-\underset{\underset{O}{\|}}{C}-OC_{16}H_{33}$

Composé B : $C_{16}H_{33}-O-\underset{\underset{O}{\|}}{C}-NH-(CH_2)_{10}-\underset{\underset{O}{\|}}{C}-OH$

Composé C : $C_{10}H_{21}-O-\underset{\underset{O}{\|}}{C}-NH-(CH_2)_{10}-\underset{\underset{O}{\|}}{C}-OH$

Composé D : $C_{16}H_{33}-O-\underset{\underset{O}{\|}}{C}-NH-(CH_2)_{10}-\underset{\underset{O}{\|}}{C}-OC_8H_{17}$

Composé E : $C_{16}H_{33}-O-\underset{\underset{O}{\|}}{C}-NH-(CH_2)_{10}-\underset{\underset{O}{\|}}{C}-OC_2H_5$

Composé F : $C_{10}H_{21}-O-\underset{\underset{O}{\|}}{C}-NH-(CH_2)_{10}-\underset{\underset{O}{\|}}{C}-OC_{12}H_{25}$

Composé G : $C_{10}H_{21}-O-\underset{\underset{O}{\|}}{C}-NH-(CH_2)_{10}-\underset{\underset{O}{\|}}{C}-OC_8H_{17}$

A partir de chacun des composés ci-dessus on a préparé une composition aérosol ayant la composition suivante :

| | |
|---|---|
| Composé à étudier .......................... | 1,5 % |
| Octyl-2-dodécanol .......................... | 20,0 % |
| Myristate d'isopropyle...................... | 20,0 % |
| Trioléate de sorbitan oxyéthyléné à l'aide de 20 moles d'oxyde d'éthylène ................. | 4,0 % |
| Oléate de sorbitan oxyéthyléné à l'aide de 40 moles d'oxyde d'éthylène ................. | 3,0 % |
| Parfum...................................... | 0,3 % |
| Huile de vaseline........................... | 51,2 % |

On introduit 47 g de la composition obtenue dans un récipient aérosol en aluminium monobloc puis après avoir serti le récipient avec une valve muni d'un tube plongeur, on met sous pression à l'aide de 3 g de 1,1-difluoroéthane.

Protocole des tests

Le composé à étudier doit donner une mousse ayant une masse volumique, après expansion à l'air à partir d'un dispositif aérosol, inférieure ou égale à 0,3 g/cm$^3$ à 20 °C, et doit être d'une stabilité supérieure à 30 secondes et plus avantageusement supérieure ou égale à 3 min.

La masse volumique de la mousse est mesurée selon la méthode suivante :

24 heures après pressurisation des aérosols en salle conditionnée à 20 °C ± 1 °C, on remplit de la mousse produite par chaque aérosol, un godet cylindrique gradué dans sa hauteur (par 10 graduations), celui-ci ayant été préalablement pesé à vide (soit $P_1$ son poids). Chaque bombe aérosol est bien agitée avant l'emploi de façon à bien émulsionner le gaz propulseur.

Pour une répartition uniforme de la mousse dans le godet, les bombes aérosols sont utilisées avec un mouvement tournant et régulier.

Dès la fin de l'expansion de la mousse, on élimine immédiatement et rapidement à l'aide d'une spatule large la mousse dépassant du godet et l'on pèse à nouveau le godet (soit $P_2$ son poids).

On détermine alors la masse volumique de la mousse selon la formule suivante :

$$\text{Masse volumique à } 20°C = \frac{P_2 - P_1}{V}$$

V étant le volume du godet.

On effectue pour chaque bombe aérosol trois déterminations de la masse volumique et la valeur obtenue est la moyenne de ces trois déterminations (en $g/cm^3$).

A intervalles de temps réguliers, on note la chute de la mousse par le nombre de graduations visibles sur le godet.

La stabilité de la mousse est jugée très bonne lorsque aucune graduation n'est visible avant 3 min. et mauvaise lorsque au moins une graduation apparaît après 30 secondes.

Les résultats obtenus sont rassemblés dans le tableau suivant :

TABLEAU 1

| COMPOSES | A | B | C | D | E | F | G |
|---|---|---|---|---|---|---|---|
| Masse volumique $g/cm^3$ | 0,1 | 0,135 | 0,142 | 0,12 | 0,148 | 1,112 | 0,154 |
| Graduations visibles après : | | | | | | | |
| 30" | 0 | 0 | 0 | 7 | 3 | 7 | 5 |
| 45" | 0 | 0 | 0 | 10 | 7 | 9 | 7 |
| 1min | 0 | 0 | 0 | 10 | 10 | 10 | 8 |
| 1min1/2 | 0 | 0 | 0 | 10 | 10 | 10 | 10 |
| 2min | 0 | 0 | 0 | 10 | 10 | 10 | 10 |
| 3min | 0 | 0 | 1 | 10 | 10 | 10 | 10 |

Comme le montrent les résultats obtenus, il n'y a pas de différence notable dans la masse volumique des mousses obtenues, exception faite du composé F, mais par contre en ce qui concerne la stabilité, on peut constater que les mousses obtenues à l'aide des composés A, B et C sont particulièrement stables dans le temps ce qui n'est pas le cas des mousses obtenues à l'aide des composés de référence D à G.

On va maintenant donner à titre d'illustration des exemples de préparation des composés de formule générale (I) ainsi que plusieurs exemples de compositions cosmétiques anhydres sous forme d'aérosol.

EXEMPLE I

Préparation de l'acide N-carbohexadécyloxyamino-11 undécanoïque(Formule I dans laquelle R = H et $R_1$ = $C_{16}H_{33}$).

40,2 g d'acide amino-11 undécanoïque (0,2 mole) sont solubilisés dans un mélange de 450ml d'acétone et 200ml d'une solution 1N de soude. Puis simultanément, on additionne 60,9 g de chloroformiate d'hexadécyle (0,2 mole) et 200 ml d'une solution 1N de soude. Un précipité blanc apparaît progressivement dans le milieu réactionnel, qu'on laisse agiter pendant 5 heures. Le milieu réactionnel est ensuite filtré et le précipité blanc obtenu est lavé à l'eau. Le sel alcalin obtenu est essoré sous vide puis dissous et acidifié à chaud dans 650 ml d'acide acétique. Le retour à la température ambiante entraîne l'apparition d'un précipité blanc, qui est filtré et lavé avec de l'acétone.

L'acide obtenu est séché sous vide à 40 ° C jusqu'à poids constant > à 79 g (Rdt ≧ 85 %).

Point de fusion = 100 ° C ± 1 ° C.

Le spectre $RMN^{13}C$ est conforme à la structure attendue.

Spectre I.R : $1538^{cm^{-1}}$ et $1679^{cm^{-1}}$ (carbamate).

Analyse centésimale : $C_{28}H_{55}NO_4$

| | C % | H % | N % | O % |
|---|---|---|---|---|
| Calc : | 71,59 | 11,80 | 2,98 | 13,62 |
| Tr : | 71,40 | 11,84 | 2,93 | 13,68 |

EXEMPLE II

Préparation de l'acide N-carbodécyloxy amino-11 undécanoïque (Formule I dans laquelle R = H et $R_1$ = $C_{10}H_{21}$).

Selon le même mode opératoire que décrit ci-dessus à l'exemple I, et en utilisant 44,1 g de chloroformiate de décyle, on obtient 61 g de cristaux blancs (Rdt = 80 %).

Point de fusion = 91 ° C.

Le spectre $RMN^{1}H$ est conforme à la structure attendue.

Spectre I.R : $1679^{cm^{-1}}$ et $1535^{cm^{-1}}$ (carbamate).

EXEMPLE III

Préparation du N-carbohexadécyloxy amino-11 undécanoate d'hexadécyle(Formule I dans laquelle R = $C_{16}H_{33}$ et $R_1$ = $C_{16}H_{33}$).

(i) A 5 g d'acide N-carbohexadécyloxy amino-11 undécanoïque obtenu à l'exemple I ci-dessus, on ajoute 2,7 g d'hexadécanol, 300 mg d'acide paratoluène sulfonique et 120 ml de toluène. La déshydratation est effectuée par un entraînement azéotropique pendant 16 heures, puis le milieu réactionnel est évaporé à sec, sous vide.

Le précipité blanc obtenu est chromatographié sur colonne de silice (éluant : chlorure de méthylène).

Après évaporation du solvant d'élution sous vide, on récupère 5,9 g de cristaux blancs purs (Rdt = 80 %).

Point de fusion = 76 ° C.

Le spectre $RMN^{13}C$ est conforme à la structure attendue.

Spectre I.R : $1730^{cm^{-1}}$ (ester)

$1685^{cm^{-1}}$ et $1536^{cm^{-1}}$ (carbamate).

Analyse centésimale : $C_{44}H_{87}NO_4$

| | C % | H % | N % | O % |
|---|---|---|---|---|
| Calc : | 76,13 | 12,63 | 2,02 | 9,22 |
| Tr : | 75,55 | 12,75 | 2,06 | 9,76 |

(ii) A 5 g du carboxylate de sodium, obtenu intermédiairement à l'exemple I, on ajoute 3,4 g de bromo-1-hexadécyle, dans 100 ml d'acétonitrile. Après avoir laissé 8 heures à reflux, le milieu réactionnel est évaporé à sec. Le précipité blanc obtenu est chromatographié sur colonne de silice (éluant : chlorure de méthylène).

Après évaporation du solvant d'élution sous vide, on récupère 4,6 g (Rdt = 65 %) de cristaux blancs purs identiques à ceux obtenus ci-dessus en (i).

EXEMPLES DE COMPOSITIONS AEROSOL

EXEMPLE 1

On prépare selon l'invention une mousse aérosol démaquillante en procédant, dans un premier temps, au mélange des ingrédients suivants :

- Oléate de Sorbitan........................... 3,0 %
- Oléate de Sorbitan oxyéthyléné à l'aide de 40 moles d'oxyde d'éthylène.................. 2,0 %
- Trioléate de Sorbitan oxyéthyléné à l'aide de 20 moles d'oxyde d'éthylène.................. 7,0 %
- Myristate d'isopropyle...................... 20,0 %
- Huile de vaseline........................... 67,5 %
- N-carbohexadécyloxy amino-11 undécanoate d'hexadécyle ............................ 0,2 %
- Parfum...................................... 0,3 %

On introduit ensuite 95 % de la composition obtenue dans un récipient aérosol, puis après avoir serti le récipient, on met sous pression à l'aide de 5 % de 1,1-difluoroéthane comme propulseur.

On obtient une mousse légère de très bonne stabilité dans le temps (supérieure à 4 min).

EXEMPLE 2

On prépare selon l'invention une mousse aérosol démaquillante en procédant, dans un premier temps, au mélange des ingrédients suivants :

| | |
|---|---|
| - Oléate de Sorbitan oxyéthyléné à l'aide de 40 moles d'oxyde d'éthylène | 6,0 % |
| - Trioléate de Sorbitan oxyéthyléné à l'aide de 20 moles d'oxyde d'éthylène | 12,0 % |
| - Huile de vaseline | 51,5 % |
| - N-carbohexadécyloxy amino-11 undécanoate d'hexadécyle | 5,0 % |
| - Parfum | 0,5 % |
| - Lécithine de Jaune d'oeuf | 5,0 % |
| - Huile de tournesol | 20,0 % |

On introduit ensuite 95 % de la composition obtenue dans un récipient aérosol, puis après avoir serti le récipient, on met sous pression à l'aide de 5 % de 1,1-difluoroéthane comme propulseur.

La mousse obtenue est compacte, serrée et fine. Elle est très agréable à l'application et s'élimine facilement par application d'eau sur le visage.

EXEMPLE 3

On prépare selon l'invention une mousse aérosol démaquillante en procédant, dans un premier temps, au mélange des ingrédients suivants :

| | |
|---|---|
| - Oléate de Sorbitan oxyéthyléné | 3,0 % |
| - Trioléate de Sorbitan | 4,0 % |
| - Octyl-2-dodécanol | 20,0 % |
| - Myristate d'isopropyle | 20,0 % |
| - N-carbohexadécyloxyamino-11 undécanoïque | 1,5 % |
| - Parfum | 0,3 % |
| - Huile de vaseline | 51,2 % |

On introduit ensuite 94 % de la composition obtenue dans un récipient aérosol, puis après avoir serti le récipient, on met sous pression à l'aide de 6 % de dioxyde de carbone comme propulseur.

Dans cet exemple, l'acide N-carbohexadécyloxyamino-11 undécanoïque peut être remplacé par la même quantité de l'acide N-carbodécyloxyamino-11 undécanoïque.

EXEMPLE 4

On prépare selon l'invention une mousse aérosol démaquillante en procédant, dans un premier temps, au mélange des ingrédients suivants :

- Huile de jojoba........................... 10 %
- Lécithine de jaune d'oeuf.................. 10 %
- Alcool isocétylique........................ 20 %
- N-carbohexadécyloxy amino-11 undécanoate d'hexadécyle.............................. 2 %
- Parfum..................................... 0,3 %
- Huile de vaseline.......................... 57,7 %

On introduit ensuite 90% de la composition obtenue dans un récipient aérosol, puis, après avoir serti le récipient, on met sous pression à l'aide de 10 % de butane.

EXEMPLE 5

On prépare selon l'invention une mousse aérosol démaquillante en procédant dans une premier temps au mélange des ingrédients suivants :

- Huile de vaseline.......................... 20 %
- Octyl-2-dodécanol.......................... 30 %
- Tétraoléate de Sorbitan.................... 20 %
- N-carbohexadécyloxy amino-11 undécanoate d'hexadécyle.............................. 1 %
- Parfum..................................... 0,2 %
- Myristate d'isopropyle..................... 28,8 %

On introduit ensuite 92 % de la composition obtenue dans un récipient aérosol, puis après avoir serti le récipient, on met sous pression à l'aide de 8 % de 1,1-difluoroéthane.

**Revendications**

1. Composition cosmétique anhydre sous forme d'aérosol pour la formation d'une mousse, constituée d'un propulseur et d'une phase grasse contenant en association au moins une huile cosmétique ou un mélange d'une huile et d'un corps gras et au moins un agent moussant caractérisée par le fait que ledit agent moussant correspond à la formule générale suivante :

$$R_1 - O - \underset{\underset{O}{\|}}{C} - NH - (CH_2)_{10} - \underset{\underset{O}{\|}}{C} - OR \qquad (I)$$

dans laquelle
R représente un atome d'hydrogène ou un radical alkyle ayant de 14 à 20 atomes de carbone, et
$R_1$ représente un radical alkyle ayant de 8 à 18 atomes de carbone.

2. Composition selon la revendication 1, caractérisée par le fait que le radical R représente un radical alkyle ayant de 16 à 18 atomes de carbone et que le radical $R_1$ représente un radical alkyle ayant de 10 à 16 atomes de carbone.

**3.** Composition selon la revendication 1 ou 2, caractérisée par le fait que les radicaux alkyles ayant de 14 à 20 atomes de carbone sont choisis parmi les suivants : tétradécyle, hexadécyle et octadécyl.

**4.** Composition selon l'une des revendications 1 et 2, caractérisée par le fait que les radicaux alkyles ayant de 8 à 18 atomes de carbone sont choisis parmi les suivants : octyle, décyle, dodécyle, tétradécyle, hexadécyle, hexyl-2-décyle et isostéaryle.

**5.** Composition selon l'une quelconque des revendications précédentes, caractérisée par le fait que l'agent moussant correspondant à la formule générale (I) est choisi parmi :
l'acide N-carbohexadécyloxy amino-11 undécanoïque,
l'acide N-carbodécyloxy amino-11 undécanoïque,
le N-carbohexadécyloxy amino-11 undécanoate d'hexadécyle,
le N-carbohexadécyloxy amino-11 undécanoate d'octadécyle, et
le N-carbodécyloxy amino-11 undécanoate d'hexadécyle.

**6.** Composition selon l'une quelconque des revendications précédentes, caractérisée par le fait que la phase grasse contient de 20 à 99,95 % et de préférence de 25 à 85 % d'au moins une huile ou d'un mélange d'une huile ou d'un corps gras par rapport au poids total de ladite phase.

**7.** Composition selon l'une quelconque des revendications précédentes, caractérisée par le fait que l'agent moussant de formule générale (I) est présent dans la phase grasse en une proportion comprise entre 0,05 et 20 % en poids et de préférence entre 0,2 et 5 % en poids par rapport au poids total de ladite phase.

**8.** Composition selon l'une quelconque des revendications précédentes, caractérisée par le fait que la phase grasse contient en outre au moins un agent tensioactif oléosoluble du type nonionique.

**9.** Composition selon la revendication 8, caractérisée par le fait que l'agent tensioactif est présent à une concentration comprise entre 5 et 60 % en poids par rapport au poids de la phase grasse.

**10.** Composition selon l'une quelconque des revendications précédentes, caractérisée par le fait que le propulseur est présent à une concentration comprise entre 1 et 20 % en poids et de préférence entre 3 et 15 % en poids par rapport au poids total de la composition.

**11.** Composition selon l'une quelconque des revendications précédentes, caractérisée par le fait que le propulseur est choisi parmi le dioxyde de carbone, le protoxyde d'azote, le propane, les butanes (isobutane), le pentane, l'isopentane, le néopentane et leurs mélanges ainsi que par des hydrocarbures halogénés choisis parmi le 1,1-difluoroéthane, le dichlorotétrafluoroéthane, le monochlorodifluorométhane, le dichlorodifluorométhane, le monochlorodifluoroéthane et les mélanges desdits propulseurs.

**12.** Composition selon l'une quelconque des revendications précédentes, caractérisée par le fait qu'elle contient en outre au moins un ingrédient cosmétique choisi parmi les vitamines, les extraits végétaux ou animaux, les agents conservateurs et les parfums.

## Claims

**1.** Anhydrous cosmetic composition in the form of aerosol for forming a foam, consisting of a propellant and of a fatty phase containing, used in combination, at least one cosmetic oil or a mixture of an oil and of a fatty substance and at least one foaming agent, characterized in that the said foaming agent corresponds to the following general formula:

$$R_1 - O - \underset{\underset{O}{\|}}{C} - NH - (CH_2)_{10} - \underset{\underset{O}{\|}}{C} - OR \qquad (I)$$

in which
R denotes a hydrogen atom or an alkyl radical containing from 14 to 20 carbon atoms, and

$R_1$ denotes an alkyl radical containing from 8 to 18 carbon atoms.

**2.** Composition according to Claim 1, characterized in that the radical R denotes an alkyl radical containing from 16 to 18 carbon atoms and that the radical $R_1$ denotes an alkyl radical containing from 10 to 16 carbon atoms.

**3.** Composition according to Claim 1 or 2, characterized in that the alkyl radicals containing from 14 to 20 carbon atoms are chosen from the following: tetradecyl, hexadecyl and octadecyl.

**4.** Composition according to either of Claims 1 and 2, characterized in that the alkyl radicals containing from 8 to 18 carbon atoms are chosen from the following: octyl, decyl, dodecyl, tetradecyl, hexadecyl, 2-hexyldecyl and isostearyl.

**5.** Composition according to any one of the preceding claims, characterized in that the foaming agent corresponding to the general formula (I) is chosen from:
N-hexadecyloxycarbonyl-11-aminoundecanoic acid,
N-decyloxycarbonyl-11-aminoundecanoic acid,
hexadecyl N-hexadecyloxycarbonyl-11-aminoundecanoate,
octadecyl N-hexadecyloxycarbonyl-11-aminoundecanoate and
hexadecyl N-decyloxycarbonyl-11-aminoundecanoate.

**6.** Composition according to any one of the preceding claims, characterized in that the fatty phase contains from 20 to 99.95 % and preferably from 25 to 85 % of at least one oil or of a mixture of an oil or of a fatty substance, relative to the total weight of the said phase.

**7.** Composition according to any one of the preceding claims, characterized in that the foaming agent of general formula (I) is present in the fatty phase in a proportion of between 0.05 and 20 % by weight and preferably between 0.2 and 5 % by weight relative to the total weight of the said phase.

**8.** Composition according to any one of the preceding claims, characterized in that the fatty phase additionally contains at least one oil-soluble surface-active agent of the nonionic type.

**9.** Composition according to Claim 8, characterized in that the surface-active agent is present in a concentration of between 5 and 60 % by weight relative to the weight of the fatty phase.

**10.** Composition according to any one of the preceding claims, characterized in that the propellant is present in a concentration of between 1 and 20 % by weight and preferably between 3 and 15 % by weight relative to the total weight of the composition.

**11.** Composition according to any one of the preceding claims, characterized in that the propellant is chosen from carbon dioxide, nitrous oxide, propane, butanes (isobutane), pentane, isopentane, neopentane and their mixtures and from halogenated hydrocarbons chosen from 1,1-difluoroethane, dichlorotetrafluoroethane, monochlorodifluoromethane, dichlorodifluoromethane, monochlorodifluoroethane and mixtures of the said propellants.

**12.** Composition according to any one of the preceding claims, characterized in that it additionally contains at least one cosmetic ingredient chosen from vitamins, plant or animal extracts, preserving agents and perfumes.

## Patentansprüche

**1.** Wasserfreie, kosmetische Zubereitung in Form eines Aerosols zur Bildung eines Schaumes, bestehend aus einem Treibmittel und einer Fettphase, die eine Mischung aus mindetens einem kosmetischen Öl oder einem Gemisch eines Öl und eines Fettgrundstoffs mit mindestens einem Schäumungsmittel enthält, welche dadurch gekennzeichnet ist, daß das Schäumungsmittel der folgenden allgemeinen Formel entspricht:

$$(I)\quad R_1 - O - \underset{\underset{O}{\|}}{C} - NH - (CH_2)_{10} - \underset{\underset{O}{\|}}{C} - OR$$

in der
R ein Wasserstoffatom oder ein Alkylradikal mit 14 bis 20 Kohlenstoffatomen und
$R_1$ ein Alkylradikal mit 8 bis 18 Kohlenstoffatomen darstellen.

**2.** Zubereitung gemäß Anspruch 1, dadurch gekennzeichnet, daß der Rest R ein Alkylradikal mit 16 bis 18 Kohlenstoffatomen und der Rest $R_1$ ein Alkylradikal mit 10 bis 16 Kohlenstoffatomen bedeuten.

**3.** Zubereitung gemäß Anspruch 1 oder 2, dadurch gekennzeichnet, daß die Alkylradikale mit 14 bis 20 Kohlenstoffen aus Tetradecyl, Hexadecyl und Octadecyl ausgewählt sind.

**4.** Zubereitung gemäß einem der Ansprüche 1 und 2, dadurch gekennzeichnet, daß die Alkylradikale mit 8 bis 18 Kohlenstoffatomen aus Octyl, Decyl, Dodecyl, Tetradecyl, Hexadecyl, Hexyl-2-decyl und Isostearyl ausgewählt sind.

**5.** Zubereitung gemäß einem der vorstehenden Ansprüche, dadurch gekennzeichnet, daß das der allgemeinen Formel (I) entsprechende Schäumungsmittel aus
N-Hexadecyloxycarbonyl-11-aminoundecansäure,
N-Decyloxycarbonyl-11-aminodecansäure,
N-Hexadecyloxycarbonyl-11-aminoundecansäure-hexadecylester,
N-Hexadecyloxycarbonyl-11-aminoundecansäure-octadecylester und
N-Decyloxycarbonyl-11-aminoundecansäure-hexadecylester
ausgewählt ist.

**6.** Zubereitung gemäß einem der vorstehenden Ansprüche, dadurch gekennzeichnet, daß die Fettphase 20 bis 99,95 % und vorzugsweise 25 bis 85 % mindestens eines Öls oder eines Gemischs eines Öls und eines Fettgrundstoffes enthält, bezogen auf das Gesamtgewicht der Phase.

**7.** Zubereitung gemäß einem der vorstehenden Ansprüche, dadurch gekennzeichnet, daß das Schäumungsmittel der Formel (I) in der Fettphase in einer Menge von 0,05 bis 20 Gew.-% und vorzugsweise von 0,2 bis 5 Gew.-% enthalten ist, bezogen auf das Gesamtgewicht der Phase.

**8.** Zubereitung gemäß einem der vorstehenden Ansprüche, dadurch gekennzeichnet, daß die Fettphase zusätzlich mindestens ein in Öl lösliches Tensid vom nicht-ionischen Typ enthält.

**9.** Zubereitung gemäß Anspruch 8, dadurch gekennzeichnet, daß das Tensid in einer Konzentration von 5 bis 60 Gew.-% enthalten ist, bezogen auf das Gesamtgewicht der Fettphase.

**10.** Zubereitung gemäß einem der vorstehenden Ansprüche, dadurch gekennzeichnet, daß das Treibmittel in einer Konzentration von 1 bis 20 Gew.-% und vorzugsweise von 3 bis 15 Gew.-% vorhanden ist, bezogen auf das Gesamtgewicht der Zubereitung.

**11.** Zubereitung gemäß einem der vorstehenden Ansprüche, dadurch gekennzeichnet, daß das Treibmittel ausgewählt ist aus Kohlendioxid, Distickstoffmonoxid, Propan, den Butanen (Isobutan), Pentan, Isopentan, Neopentan und deren Gemischen sowie aus den halogenierten Kohlenwasserstoffen, welche aus 1,1-Difluorethan, Dichlortetrafluorethan, Monochlordifluormethan, Dichlordifluormethan, Monochlordifluorethan sowie Gemischen aus diesen Treibmitteln ausgewählt sind.

**12.** Zubereitung gemäß einem der vorstehenden Ansprüche, dadurch gekennzeichnet, daß sie darüber hinaus mindestens einen kosmetischen Inhaltsstoff enthält, der aus den Vitaminen, pflazlichen oder tierischen Extrakten, Konservierungsmitteln und Parfümen ausgewählt ist.